# EUROPEAN PATENT APPLICATION

(11) **EP 1 693 671 A1**
(43) Date of publication of application: **23.08.2006**
(21) Application number: 04793403.9
(22) Date of filing: 29.10.2004
(51) Int. Cl.: G01N 33/53, G01N 33/68

(54) **METHOD OF DIAGNOSING ALZHEIMER'S DISEASE**

(30) Priority: 29.10.2003 US 515333 P
(71) Applicant: Eisai Co., Ltd., Tokyo 112-88 (JP)
(72) Inventor: KANAI, Yoshiyuki, Tokyo 1660012 (JP); YAMADA, Yuji, c/o Tsukuba Research Laboratories, Tsukuba-shi, Ibaraki 3002635 (JP); USAMI, Michinori c/o Tsukuba Research Laboratories, Tsukuba-shi, Ibaraki 3002635 (JP)
(74) Representative: Woods, Geoffrey Corlett
(86) International application number: PCT/JP2004/016483
(87) International publication number: WO 2005/040798

(57) **Abstract**

The present invention provides a detection method for a nervous system disease, which comprises reacting poly ADP-ribose and/or histone H1 with a biological sample to detect an antibody against poly ADP-ribose and/or an antibody against histone H1.

## Description

### TECHNICAL FIELD

The present invention relates to methods for detecting and diagnosing Alzheimer's disease.

### BACKGROUND ART

Alzheimer's disease (AD) and senile dementia of the Alzheimer's type (SDAT) are diseases which start to affect people in their fifties; and whose onset frequency increases with aging. In particular, since Japan will enter an aging society in 2010 where one-fourth of all the people in Japan will be over 70 years of age, AD/SDAT cases are expected to increase, and advanced stages of these diseases will lead to a reduction in national productivity and a serious increase in the burden of medical expenses. Thus, there is an urgent necessity to prevent the progression of symptoms by early diagnosis. To this end, it is important to find symptom-specific markers in blood or cerebrospinal fluid and to precisely measure the markers.

To find symptom-specific markers, many attempts have been made. Substances for this purpose include neurofilament heavy chain, tubulin, glial fibrilary acidic protein, S 100 protein, tau protein, beta amyloid precursor peptide, myelin basic protein, and heparan sulfate proteoglycan. Further attempts have also been made to search, e.g., autoantibodies against these substances [Terryberry JW, Thor G, Peter JB. Autoantibodies in neurodegenerative diseases: antigen-specific frequencies and intrathecal analysis. Neurobiol Aging 1998; 19: 205-216.]. These searches are not ineffective, but lack specificity; they are difficult to achieve highly sensitive detection. In view of the foregoing, there has been a strong demand for the development of a simple and highly specific diagnostic method for AD and SDAT.

### DISCLOSURE OF THE INVENTION

The object of the present invention is to provide a method for specifically detecting brain nervous system diseases such as Alzheimer's disease and senile dementia of the Alzheimer's type, as well as a diagnostic method for these diseases.

As a result of extensive and intensive efforts made to achieve the object stated above, the inventors of the present invention have found that the above diseases can be detected by measuring the blood levels of anti-histone H1 and anti-poly ADP-ribose antibodies in patients with Alzheimer's disease, etc. This finding led to the completion of the present invention.

Namely, the present invention is directed to the following.
(1) A detection method for a nervous system disease, which comprises reacting poly ADP-ribose and/or histone H1 with a biological sample to detect an antibody against poly ADP-ribose and/or an antibody against histone H1.
   In the present invention, a body fluid such as blood, saliva, serosity, lymph or serum may be used as a biological sample. Likewise, examples of a nervous system disease include, but are not limited to, Alzheimer's disease (AD) or senile dementia of the Alzheimer's type (SDAT). Biological samples (e.g., body fluids) from AD or SDAT patients contain either or both anti-poly ADP-ribose antibody (also referred to as "anti-pADPR antibody") and anti-histone H1 antibody (also referred to as "anti-H1 antibody"), and these autoantibodies are of both IgG1 and IgG2 subclasses. In contrast, in patients with systemic lupus erythematosus (SLE), which is a representative autoimmune disease, their autoantibodies are predominantly of the IgG2 subclass. Thus, when the ratio between IgG1 and IgG2 (G1/G2 ratio) is measured, the resulting value can be used as an index to determine whether a test patient suffers from AD or SDAT. Moreover, such a measurement also allows reclassification (subtyping) of these diseases. Likewise, when anti-pADPR IgA and/or anti-H1 IgA is measured in addition to anti-pADPR IgG and/or anti-histone H1 IgG, the resulting value can also be used as an index to determine whether a test patient suffers from AD or SDAT.
(2) A diagnostic method for a nervous system disease, which comprises reacting poly ADP-ribose and/or histone H1 with a biological sample to detect an antibody against poly ADP-ribose and/or an antibody against histone H1.
   Details of the biological sample and the nervous system disease in this embodiment are the same as described for the invention in (1) above. Also in the diagnostic method of the present invention, the ratio between IgG1 and IgG2 can be used as an index for diagnosis. Likewise, when anti-pADPR IgA and/or anti-H1 IgA is measured in addition to anti-pADPR IgG and/or anti-histone H1 IgG, the resulting value can also be used as an index to determine whether a test patient suffers from AD or SDAT.
(3) A diagnostic or detection kit for a nervous system disease, which comprises poly ADP-ribose and/or histone H1.
(4) A diagnostic or detection plate for a nervous system disease, which comprises poly ADP-ribose and/or histone H1 immobilized on a solid phase.
   In the above kit and plate, the nervous system disease is, for example, Alzheimer's disease or senile dementia of the Alzheimer's type.
(5) A method for solid-phasing histone H1 onto a solid phase, which comprises diluting histone H1 with a solution of 0.25 M NaCl and 25 mM Tris buffer (pH 7.4) and then immobilizing the same on the solid phase.

### BRIEF DESCRIPTION OF DRAWINGS

Figure 1 shows the structure of ADP-ribose which is a unit of poly ADP-ribose.
Figure 2 shows the detection results of anti-pADPR and anti-H1 antibodies in AD and SDAT patients.
Figure 3 shows the detection results of anti-pADPR and anti-H1 antibodies in SLE patients.
Figure 4 shows the correlation between anti-H1 antibody and anti-pADPR antibody in AD and SDAT patients.
Figure 5 shows the correlation between anti-H1 antibody and anti-pADPR antibody in SLE patients.
Figure 6 shows the detection results of anti-pADPR IgG and anti-pADPR IgA in AD patients and healthy individuals.

### BEST MODE FOR CARRYING OUT THE INVENTION

### 1. Summary

Recently, there have been reports of increased ADP-ribosylation activity in the cell nuclei of brain neurocytes, especially macroglia cells (astrocytes), in AD patients [Increased poly (ADP-ribosyl) action of nuclear proteins in Alzheimer's disease. Brain 1999; 122: 247-253] and high level expression of histone H1 in the macroglia cell membrane [Bolton SJ, Russelakis-Carneiro M, Betmouni S, Perry, VH. Non-nuclear histone H1 is upregulated in neurons and astrocytes in prion and Alzheimer's diseases but not in acute neurodegeneration. Neuropathol Applied Neurobiol 1999; 25: 425-432.]. It is known that these two phenomena are closely related to each other. ADP-ribosylation is a phenomenon occurring predominantly in proteins [Hayaishi O, Ueda K. Poly (ADP-ribose) and ADP-ribosylation of proteins. Ann Rev Biochem 1977; 46: 95-116] and is also known as a protein modification reaction. A representative protein to be modified (i.e., ADP-ribosylated) is histone H1. H1 plays an important role in the condensation of nuclear chromatin and its constitutional unit nucleosome (NS). When H1 is ADP-ribosylated, chromatin is condensed into a tight or loose structure, depending on the degree of ribosylation [Kanai Y, Kawamitsu H, Tanaka M et al. A novel method for purification of poly (ADP-ribose). J. Biochem 1980; 88: 917-920]. This means that ADP-ribosylation of chromatin is deeply involved in the regulation of gene expression and also produces an effect of DNA repair upon DNA breakage caused by carcinogens or the like [Virag L, Suzabo C. The therapeutic potential of poly(ADP-ribose) polymerase inhibitors. Pharmaceut Rev 2002; 54: 375-429]. Moreover, in the near future, ADP-ribosylation will also become deeply involved in aging as well as in functional and morphological changes in cell nuclear chromatin.

The inventors of the present invention reported before anyone else that poly ADP-ribose (hereinafter referred to as "pADPR") synthesized by extension reaction of ADP-ribosylation allowed mice and rabbits to produce anti-pADPR antibody when bound to a basic protein through ionic bonding [Kanai Y, Miwa M, Matsushima T. Sugimura T. Studies on poly (adenosine diphosphate ribose) antibody. Biochem Biophys Res Commun 1974; 59: 300-306]. The inventors have further found that when rabbits are immunized with ADP-ribosylated histone, both anti-histone antibody and anti-pADPR antibody are produced simultaneously, and much stronger antibodies are produced than if rabbits are immunized with unmodified histone [Kanai Y, Sugimura T. Comparative studies on antibodies to poly(ADP-ribose) in rabbits and patients with systemic lupus erythematosus. Immunology 1981; 43: 101-110., Kanai Y, Sugimura T. Systemic lupus erythematosus. In: ADP-Ribosylation Reactions. Hayaishi O, Ueda K eds. Academic Press, New York 1982: pp.533-546].

In view of their research results as stated above, the inventors of the present invention have assumed that histone H1 expressed on the astrocyte membrane surface in AD patients has a high probability of being ADP-ribosylated, and hence have attempted to measure the blood levels of anti-histone H1 and anti-pADPR antibodies in AD patients.

On the other hand, aside from an immunological viewpoint, it is known that poly ADP-ribosylation is deeply involved in neurocyte repair following impaired blood flow such as ischemia in the brain [Szabo C, Dawson VD. Role of poly (ADP-ribose) synthetase in inflammation and ischemia-reperfusion. Trends Pharmacol Sci 1998; 19: 287-298]. In this case, ADP-ribosylation is overstimulated for repair of DNA damage, so that NAD is depleted to induce cell death, which in turn participates in brain degeneration. When an inhibitor of PARP (pADPR synthetase) is administered during such ischemia or impaired blood flow in the brain, neurocyte death and neurodegeneration can be prevented. Based on this finding, PARP inhibitors have been aggressively developed [Virag L, Suzabo C. The therapeutic potential of poly (ADP-ribose) polymerase inhibitors. Pharmaceut Rev 2002; 54: 375-429]. As if to support this, some reports indicate that PARP knockout mice are prevented from developing neurocyte death resulting from impaired blood flow in the brain [Ellasson MJL, Sampei K, Mandir AS et al. Poly (ADP-ribose) polymerase gene disruption renders mice resistant to cerebral ischemia. Nature Med 1997; 3: 1089-1095].

In summary, it is expected that in AD cases, ADP-ribosylation in the brain is stimulated to cause pADPR deposition in the brain. Incidentally, recent reports suggest that the target molecule of ADP-ribosylation is more frequently PARP than H1 [Lindhal T, Satoh M, Poirier GG, Klungland A. Posttranlational modification of poly(ADP-ribose) polymerase induced by DNA strand break. Trends Biochem Sci 1995; 20: 404-411]. Loosing of the brain barrier expected with aging not only leads to leakage of pADPR into the blood, but also increases the opportunity for exposure to the immune system; there are very strong theoretical grounds for studying immune responses to pADPR in AD.

### 2. Detection method for nervous system diseases

The present invention provides a detection method for a nervous system disease, which comprises reacting poly ADP-ribose and/or histone H1 with a biological sample to detect an antibody against poly ADP-ribose and/or an antibody against histone H1. Namely, this method involves detecting the level of anti-poly ADP-ribose antibody and/or anti-histone H1 antibody in a biological sample by using poly ADP-ribose and/or histone H1, thereby detecting a nervous system disease.

### (1) Nervous system diseases

As used herein, the term "nervous system disease" encompasses a wide range of diseases in the nervous system associated with ADP-ribosylation. Example includes Alzheimer's disease (AD) and senile dementia of the Alzheimer's type (SDAT). In particular, this method is useful for diseases during which the expression of anti-poly ADP-ribose antibody and/or anti-histone H1 antibody is observed.

### (2) Reaction of poly ADP-ribose and/or histone HI with a biological sample, and antibody detection

The method of the present invention involves adding and reacting a biological sample to a plate, on which poly ADP-ribose and/or histone H1 is solid-phased, to detect anti-poly ADP-ribose antibody and/or anti-histone H1 antibody in the sample.

### (a) Poly ADP-ribose (pADPR)

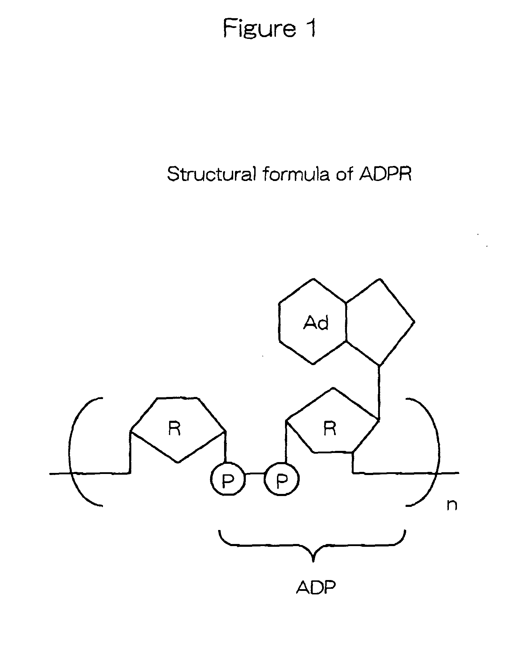

Poly ADP-ribose used in the present invention is composed of multiple ("n" in Figure 1) units of ADP-ribose (Figure 1) linked one after another. In Figure 1, "R" represents ribose, "P" represents a phosphate group, and "Ad" represents adenine. pADPR can be synthesized from nicotinamide adenine dinucleotide (NAD) or NAD⁺. For example, pADPR can be obtained by synthesis using NAD as a substrate and calf thymus nuclei as an enzyme source, and the subsequent purification [Kanai Y, Kawamitsu H, Tanaka M et al. A novel method for purification of poly(ADP-ribose). J. Biochem 1980; 88: 917-920]. The length of poly ADP-ribose used in the present invention is not limited as long as it is recognized by anti-poly ADP-ribose antibody, but the average chain length is 10 to 50, preferably 20 to 40, and more preferably 30. It should be noted that pADPR used in the present invention is preferably of high purity and the percentages of DNA and histone contained in pADPR are each preferably 1% or less.

### (b) Histone H1

Histone H1 used in the present invention can be separated and purified from total histones by an ion concentration gradient after purifying the total histones from a sample such as human-derived cells. For example, total histones may be isolated from the cell nuclei of human promyelocytic leukemia cell line HL60 and solubilized by being dialyzed against 0.25 M NaCl + 25 mM Tris buffer (pH 7.4) (Solution A), followed by treatment using a cation column HiTrap SP (Amersham Biosciences) under acidic (pH 3.5) conditions with a concentration gradient of sodium chloride (NaCl) to separate only H1 from the total histones [Kanai Y, Sugimura T. Comparative studies on antibodies to poly(ADP-ribose) in rabbits and patients with systemic lupus erythematosus. Immunology 1981; 43: 101-110.].

The present invention has succeeded in solubilizing total histones by dialysis against Solution A mentioned above. It has previously been known that histones are generally dissolved only in diluted hydrochloric acid or a salt-free aqueous solution. However, such a state resulted in a reduced adhesion rate when histones were solid-phased in the ELISA system described later. In contrast, the present invention has succeeded in increasing the adhesion efficiency of histone H1 onto a solid phase by using Solution A mentioned above. It should be noted that histone H1 used in the present invention is preferably of high purity and the content of DNA contained in histone H1 is preferably 1% or less.

### (c) Plates

pADPR obtained in (a) above and/or histone H1 obtained in (b) above may be diluted with an appropriate buffer such as Solution A mentioned above or PBS, added in an appropriate amount to microtiter plates or the like, allowed to stand overnight up to a whole day and night at 4°C, and then solid-phased by being immobilized on the solid phase (plates). The microtiter plates used for this purpose may be commercially available ones and can be selected as appropriate by those skilled in the art. For example, in the case of using 96-well Immulon 2Hb plates, 50 to 100 µl of a pADPR and/or histone H1 solution may be added.

As described above, it is preferable to use Solution A for dilution of histone H1. The present invention includes a method for solid-phasing histone H1, which comprises diluting histone H1 with Solution A (0.25 M NaCl + 25 mM Tris buffer (pH 7.4)) and then immobilizing the same on a solid phase. For example, the present invention includes a method in which histone H1 obtained as described in (b) above (i.e., histone H1 purified from total histones which have been solubilized by dialysis against Solution A) or histone H1 obtained in another manner is diluted with Solution A, and the resulting diluted solution is added to a solid phase such as a plate, so that histone H1 is solid-phased.

Next, the plates on which pADPR and/or histone H1 is solid-phased are washed with a buffer such as TBS (25 mM Tris, 140 mM NaCl, 0.04% sodium azide (NaN₃), pH 7.4) or PBS. Next, antigen-unadsorbed sites on the plates are blocked by addition of, e.g., TBS containing 1% to 5% skimmed milk or 0.5% to 3% bovine serum albumin (BSA) in an amount of 100 to 200 µl for 96-well plates, followed by reaction at room temperature for 1 hour. The plates are then washed once to three times with, e.g., TBS to prepare antigen plates for ELISA.

The present invention includes a diagnostic or detection plate for a nervous system disease (hereinafter also referred to as "the plate of the present invention"), which comprises poly ADP-ribose and/or histone H1 immobilized (solid-phased) on a solid phase. Conditions for solid-phasing, washing and blocking the plate of the present invention can be altered as appropriate by those skilled in the art and are not limited to the conditions shown above. Likewise, the plate of the present invention is not limited by the presence or absence of the above washing and blocking treatment, as long as pADPR and/or histone H1 is solid-phased on the plate.

### (d) Biological samples (analytes)

The biological sample used in the present invention is collected from a subject in whom a nervous system disease is detected. The biological sample used for this purpose may be a body fluid, more specifically blood, saliva, serosity, lymph or the like. The biological sample may also be serum.

### (e) ELISA

In the present invention, ELISA (enzyme-linked immunosorbent assay) may be used for measurement of antibody levels, by way of example.

In the present invention, the ELISA method involves first adding the biological samples obtained in (d) above to the plates of the present invention obtained in (c) above. The biological samples to be added may be diluted with TBS, PBS, physiological saline or the like before addition to the plates. The amount of biological samples to be added to the plates of the present invention is preferably 50 to 100 µl for 96-well plates. After sample addition, the plates are allowed to stand at room temperature for 1 hour. The plates are then washed once to five times with a buffer such as TBS or PBS.

Subsequently, a secondary antibody is added and reacted at room temperature for 1 hour, followed by washing the plates as described above. In the present invention, antibody dilution is 200-fold, and the solution used for dilution and reaction may be, but is not limited to, TBS containing 1% BSA, 0.4% skimmed milk and 0.02 % NaN₃. As a secondary antibody, anti-human IgG antibody may be used for detection of anti-pADPR IgG or anti-histone H1 IgG, while anti-human IgA antibody may be used for detection of anti-pADPR IgA or anti-histone H1 IgA. In particular, the anti-human IgG antibody used may be an antibody specific to the human IgG subclass G1, G2, G3 or G4. Such a secondary antibody may be labeled with biotin, avidin or HRP (horse radish peroxidase), etc.

Next, the secondary antibody is detected, e.g., by color development using a tertiary antibody or a tertiary reagent. In a case where the above biotin- or avidin-labeled anti-human antibody is used as a secondary antibody, color development may be accomplished by using alkaline phosphatase-labeled avidin or biotin as a tertiary reagent and p-nitrophenyl phosphate as a color-developing substrate. In the case of using the above HRP-labeled secondary antibody, TMB (tetramethyl benzidine) may be used as a tertiary reagent for luminescence. Alternatively, an antibody labeled with HRP or the like may also be used as a tertiary antibody for luminescence. For example, an HRP-labeled anti-mouse IgG antibody may be used when the secondary antibody is IgG of mouse origin, while an HRP anti-rat IgG antibody may be used when the secondary antibody is IgG of rat origin. Those skilled in the art will be able to select and implement a method appropriate for detection.

Signal detection may be accomplished by using a plate reader to measure the absorbance at a wavelength suitable for the system of color development or luminescence. For example, the absorbance at 405 nm may be measured when using an alkaline phosphatase-labeled system, or alternatively, the absorbance at 450 nm may be measured when using an HRP-labeled system together with sulfuric acid as a stop solution. The measured absorbance may be defined as an antibody titer.

### (6) Detection of nervous system diseases

In the detection of nervous system diseases, when using anti-human IgG or anti-human IgA antibody as a secondary antibody, the antibody titer of anti-pADPR antibody (including anti-pADPR IgG and anti-pADPR IgA) and/or anti-histone H1 antibody (including anti-histone H1 IgG and anti-histone H1 IgA) may be used as an index. In this case, if the antibody titer of anti-pADPR antibody and/or anti-histone H1 antibody is higher than the mean value, there is a high probability of nervous system diseases.

In patients with nervous system diseases (e.g., AD and SDAT), their autoantibodies (i.e., anti-pADPR antibody and/or anti-histone H1 antibody in the present invention) are predominantly of the IgG1 and IgG2 subclasses. In contrast, in patients with systemic lupus erythematosus, which is a representative autoimmune disease, their autoantibodies are predominantly of the IgG2 subclass. Thus, when the ratio between IgG1 and IgG2 (G1/G2) is measured, the resulting value can be used as an index to detect nervous system diseases as distinguished from autoimmune diseases. For example, it can be determined that a high G1/G2 value indicates a high probability of nervous system diseases, while a low G1/G2 value indicates a low probability of nervous system diseases.

### (7) Kits

The kit of the present invention comprises the plate of the present invention used for practicing the detection method for a nervous system disease. The kit of the present invention may further comprise additional elements required to practice the method of the present invention. Examples of such elements include a tube(s) for centrifuging a biological sample, a buffer for plate washing (e.g., TBS, PBS, Solution A), a solution for blocking, a secondary antibody, a solution for secondary antibody dilution, anti-pADPR antibody and/or anti-histone H1 antibody for use as a standard, a reaction solution for color development or luminescence, a solution for stopping color development, a chip(s), a tube(s), etc.

### 3. Diagnostic method for nervous system diseases

The present invention provides a diagnostic method for a nervous system disease, which comprises reacting poly ADP-ribose and/or histone H1 with a biological sample to detect an antibody against poly ADP-ribose and/or an antibody against histone H1. The diagnostic method of the present invention can provide a diagnosis based on the detection results of nervous system diseases obtained in 2. above.

In the diagnosis of nervous system diseases, when using anti-human IgG or anti-human IgA antibody as a secondary antibody, the antibody titer of anti-pADPR antibody (including anti-pADPR IgG and anti-pADPR IgA) and/or anti-histone H1 antibody (including anti-histone H1 IgG and anti-histone H1 IgA) may be used as an index. In this case, if the antibody titer of anti-pADPR antibody and/or anti-histone H1 antibody is higher than that of healthy individuals, a patient who provided the biological sample can be diagnosed as having a high probability of suffering from a nervous system disease.

Alternatively, in the diagnosis of nervous system diseases, when antibodies specific to anti-human IgG subclasses are used as secondary antibodies to measure G1/G2, the resulting value may be used as an index. For example, if the G1/G2 value is higher than that of SLE patients, a patient from whom the sample was derived can be diagnosed as having a high probability of suffering from a nervous system disease, but not SLE.

Moreover, in patients with nervous system diseases, there is a correlation between the levels of anti-H1 antibody and anti-pADPR antibody produced. This correlation means that anti-pADPR antibody production is observed in anti-H1 antibody-positive patients, and vice versa. Based on this, an attempt can be made to distinguish from SLE patients who are known to show a high titer of anti-pADPR antibody. For example, when anti-pADPR antibody is positive and anti-H1 antibody is also positive, nervous system diseases can be distinguished from SLE. It is therefore possible to detect and diagnose nervous system diseases as distinguished from SLE by detecting not only anti-pADPR antibody, but also anti-H1 antibody, or alternatively, by detecting anti-H1 antibody alone.

### EXAMPLES

The present invention will be further described in more detail in the following Examples, which are not intended to limit the scope of the invention.

### Example 1

Diagnostic method for AD or SDAT using anti-pADPR and anti-H1 antibodies

### 1-1. Method

### (1) Synthesis and purification of pADPR

According to the method developed by the inventors of the present invention [Kanai Y, Kawamitsu H, Tanaka M et al. A novel method for purification of poly(ADP-ribose). J. Biochem 1980; 88: 917-920], pADPR was synthesized and purified using calf thymus nuclei as an enzyme source and nicotinamide adenine dinucleotide (NAD) as a substrate. The average chain length of pADPR was set to 30. The percentages of DNA and histone contained in the polymer purified by this procedure are each 1% or less.

### (2) Purification of histone H1

Total histones were purified using the method developed by the inventors of the present invention [Kanai Y, Sugimura T. Comparative studies on antibodies to poly(ADP-ribose) in rabbits and patients with systemic lupus erythematosus. Immunology 1981; 43: 101-110.].

Namely, total histones were isolated from the cell nuclei of human promyelocytic leukemia cell line HL60 and further treated using a cation column HiTrap SP (Amersham Biosciences) under acidic (pH 3.5) conditions with a concentration gradient of NaCl to separate only H1 from the total histones.

The total histones were dialyzed against 0.25M NaCl + 25 mM Tris buffer (pH 7.4) (Solution A) and successfully solubilized. The content of DNA contained in the resulting H1 preparation was 1% or less.

### (3) Procedures for anti-pADPR antibody measurement

Anti-pADPR antibody was measured by enzyme-linked immunosorbent assay (ELISA).

Immulon 2HB microtiter plates were used as a solid phase, and pADPR was diluted with Solution A to a concentration of 1 µg/ml, added to each well in an amount of 50 µl and allowed to stand at 4°C for a whole day and night. Subsequently, the plates were washed with TBS (25 mM Tris, 140 mM NaCl, 0.04% sodium azide (NaN₃), pH 7.4) and then reacted with TBS containing 2% skimmed milk at room temperature for 1 hour to block their antigen-unadsorbed sites. Finally, the plates were washed three times with TBS to prepare antigen plates for ELISA.

Serum dilution was 200-fold, and the solution used for dilution and reaction was TBS containing 1% bovine serum albumin (BSA), 0.4% skimmed milk and 0.02% NaN₃.

As secondary antibodies, biotin-labeled anti-human subclass (G1, G2, G3, G4)-specific antibodies (Zymed) were used. Likewise, alkaline phosphatase-labeled avidin (Zymed) was used as a tertiary reagent and p-nitrophenyl phosphate was used as a color-developing substrate. The antibody titer was expressed as A405.

### (4) Procedures for anti-H1 antibody measurement

Anti-H1 antibody was measured according to the procedures for anti-pADPR antibody measurement, except that the antigen used was H1.

### (5) Analytes

Six cases of AD and 20 cases of SDAT were used as test groups. As control groups, 59 cases of non-AD and non-SDAT elderly subjects (average age: 77 ± 7 years), 40 cases of systemic lupus erythematosus (SLE) patients, and 27 cases of healthy individuals below 60 years of age were used. Sera from the test groups and the control groups were used as analytes for ELISA. The diagnosis of AD and SDAT was made according to DMS-IV (Diagnostic and Statistical Manual of Mental Disorders, 4th ed., American Psychiatric Association, Washington, D.C., 1994), while the diagnosis of SLE was made according to the American College of Rheumatology's diagnostic criteria (Updating the American College of Rheumatology Revised Criteria for the Classification of SLE (1997)).

### 1-2. Results

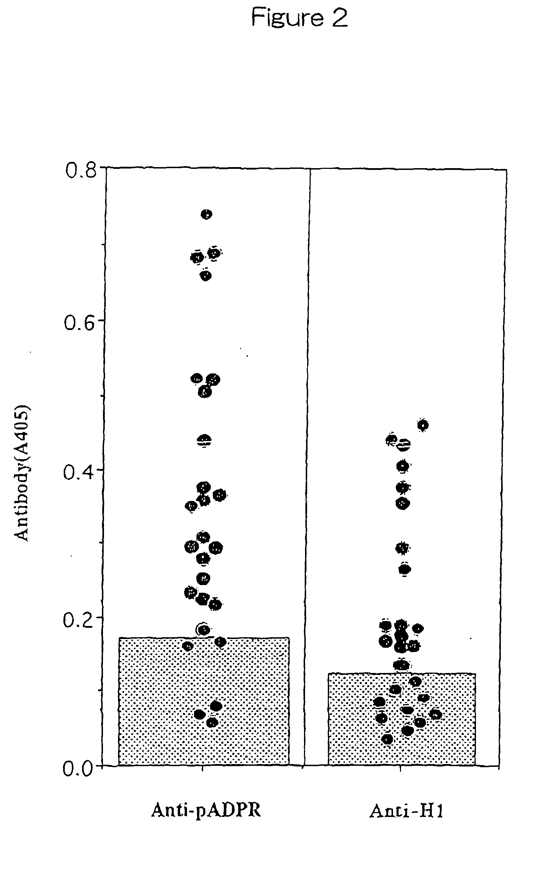

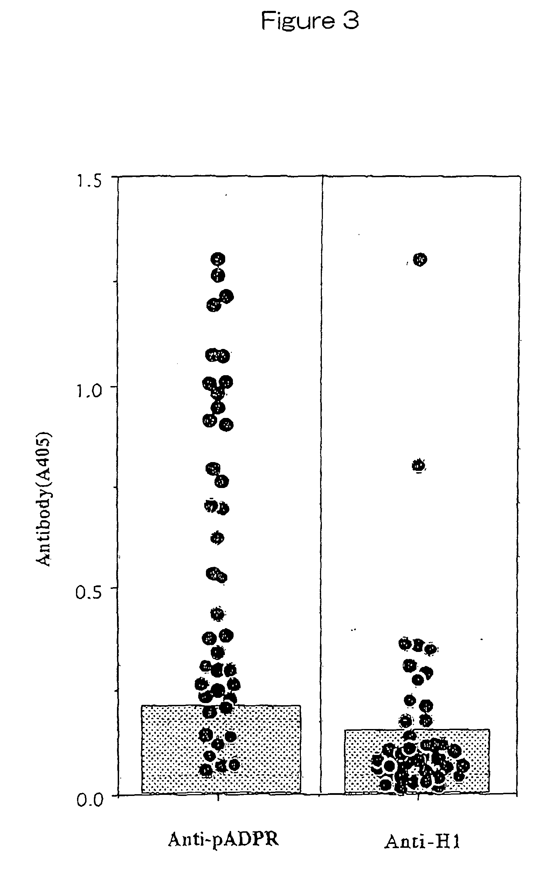

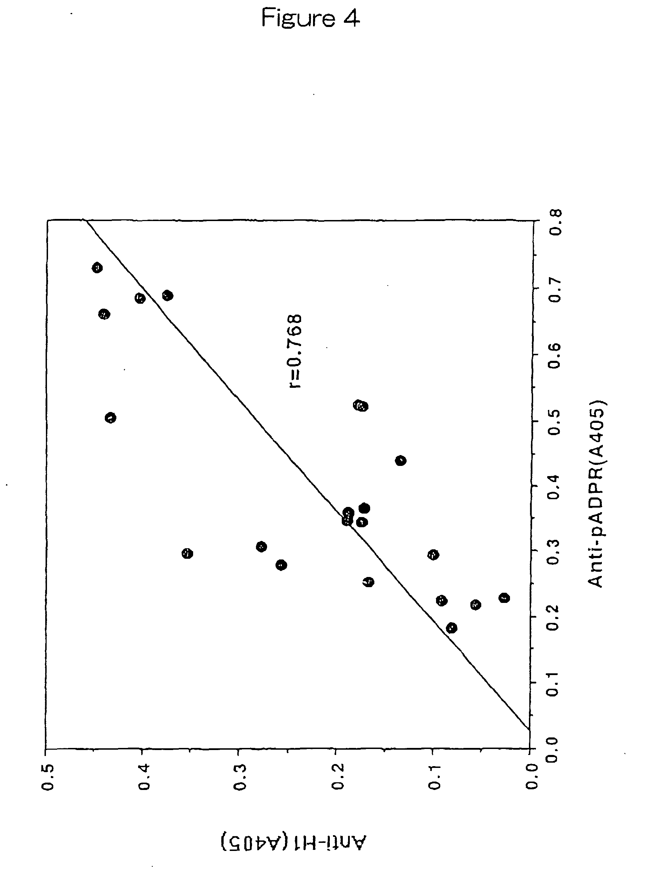

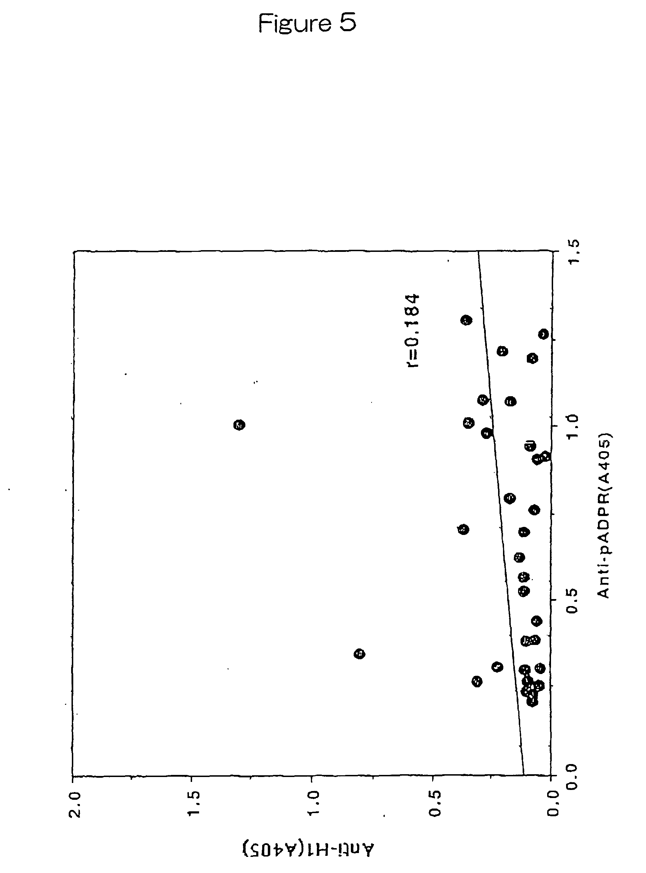

Relationship of AD with anti-pADPR antibody and anti-H1 antibody:
The results of 26 cases including both AD patients and SDAT patients are shown in Figure 2. The results obtained from the 40 cases of SLE patients are shown in Figure 3.
With respect to anti-pADPR antibody, all (100%) of the 6 AD cases showed positive results, while 15 (75%) of the 20 SDAT cases showed positive results (Figure 2, "Anti-pADPR"). Likewise, with respect to anti-H1 antibody, 50% (3/6) of the AD cases were positive, while 65% (13/20) of the SDAT cases were positive (Figure 2, "Anti-H1"). The columns in Figures 2 and 3 each represent the mean (vertical axis) and 2SD (horizontal axis) of the healthy individuals.
Moreover, among the AD and SDAT patients, those who were anti-H1-positive were all positive for anti-pADPR antibody, resulting in a correlation coefficient (r) of 0.768 (Figure 4). On the other hand, the same test was performed on the 40 cases of SLE patients known to show a high titer of anti-pADPR antibody [Kanai Y, Kawamitsu Y, Miwa M, Matsushima T, Sugimura T. Naturally-occurring antibodies to poly(ADP-ribose) in patients with systemic lupus erythematosus. Nature 1977; 265: 175-177], resulting in a correlation coefficient of 0.184, which was lower with a statistically significant difference (p<0.01) (Figures 3 and 5).
The above results not only suggest that histone H1 is modified with pADPR in the body, particularly the brain, of AD or SDAT patients, but also indicate that production mechanisms of anti-H1 and anti-pADPR antibodies in AD or SDAT patients are different from those of collagenosis SLE patients. Moreover, worthy of special note is that as a result of testing on 2 to 3 cases, autoantibodies in SLE were found to be of the IgG2 subclass, whereas autoantibodies in AD/SDAT were found to be of both IgG1 and IgG2 subclasses. This provides further support for a difference in autoantibody production mechanisms between AD/SDAT patients and SLE patients. It is therefore considered that the G1/G2 ratio will be a promising index in diagnosing nervous system diseases including AD and SDAT.

### Example 2

Diagnostic method for AD based on anti-pADPR IgA measurement

### 2-1. Method

### (1) Procedures for anti-pADPR antibody measurement

Anti-pADPR antibody was measured by enzyme-linked immunosorbent assay (ELISA).

The antigen plates for ELISA used were those prepared in Example 1(3).

The antigen plates for ELISA were washed three times with a washing solution (0.025M Tris, 0.25M NaCl, 0.1% Tween-20; pH 7.4). Into the plates, a reaction solution (0.025M Tris, 0.14M NaCl, 10% NCS; pH 7.4) and an analyte were dispensed in volumes of 50 µl/well and 5 µl/well, respectively. Before use, the analyte was diluted 21-fold from 10 µl with the reaction solution (200 µl). After shaking, the plates were reacted overnight at 4°C in a wet environment, with the plate surface being sealed. After the reaction, the plates were washed three times with the washing solution and a secondary antibody was dispensed in a volume of 50 µl/well, followed by reaction at room temperature for 60 minutes in a wet environment, with the plate surface being sealed. After the reaction, the plates were washed three times with the washing solution and TMB (3,3',5,5'-Tetramethylbenzidine; Sigma T0440) as a substrate was then dispensed in a volume of 50 µl/well, followed by reaction at room temperature for 10 to 15 minutes in the dark, with the plate surface being sealed. Subsequently, a reaction stop solution [1N H₂SO₄ (Abbott 7212)] was dispensed in a volume of 50 µl/well to stop the reaction, and the absorbance at 450 nm was measured.

### (2) Secondary antibodies

The secondary antibodies used were HRP-labeled anti-human IgG antibody (Monosan #PS104P) and HRP-labeled anti-human IgA antibody (Monosan #PS106P), each of which had been diluted 1000-fold with the reaction solution.

### (3) Analytes

For use as healthy individual sera (10 analytes), sera from persons over 50 years of age were selected among those commercially available. As AD patient sera (47 analytes), commercially available sera were used.

### 2-2. Results

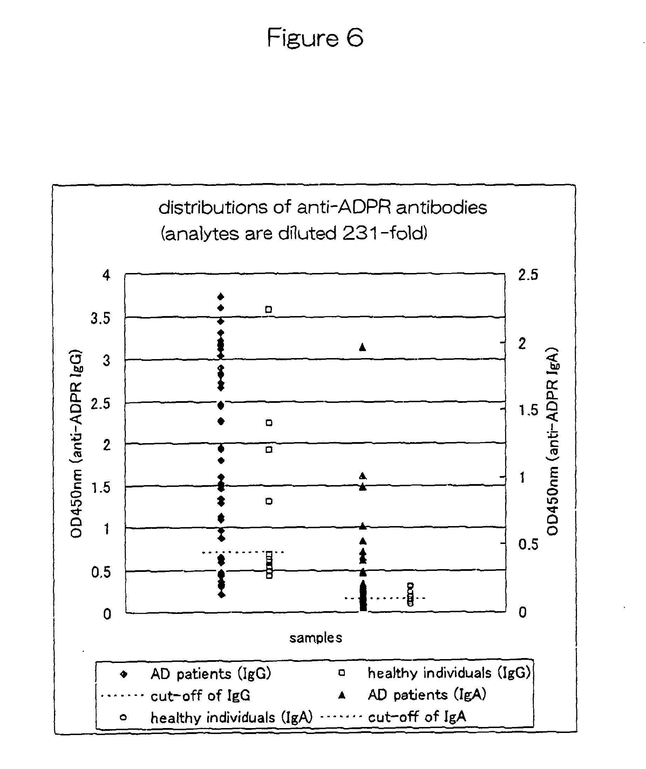

The results obtained are shown in Table 1 and Figure 6.

**Table 1**

| Accuracy of diagnosis in AD patients using anti-pADPR IgG and anti-pADPR IgA | | | | | | |
|---|---|---|---|---|---|---|
| | Cut-off | AD patients | | Healthy individuals | | Accuracy |
| | | Positive | Negative | Positive | Negative | |
| IgG measurement system | 0.7 | 34/46 | 12/46 | 4/10 | 6/10 | 40/56 |
| | | (74%) | (26%) | (40%) | (60%) | (71%) |
| IgA measurement system | 0.1 | 25/47 | 22/47 | 4/10 | 6/10 | 31/57 |
| | | (53%) | (47%) | (40%) | (60%) | (54.5%) |

### (1) IgG measurement system

When the cut-off value was set to 0.7 based on the ROC curve (receive operating characteristic curve), the detection rate was 34/46 (74%) in the AD patient sera, of which 12/46 (26%) were false negative (Table 1 and Figure 6, "AD patients (IgG)"). Likewise, 4/10 (40%) of the healthy individual sera were false positive (Table 1 and Figure 6, "Healthy individuals (IgG)"). The accuracy was 71% (Table 1, Figure 6).

### (2) IgA measurement system

In the IgA measurement system, when the cut-off value was set to 0.1 based on the ROC curve, 22/47 (46.8%) of the AD patient sera were false negative (Table 1 and Figure 6, "AD patients (IgA)") and 4/10 (40%) of the healthy individual sera were false positive (Table 1 and Figure 6, "Healthy individuals (IgA)"). The accuracy is determined to be 54.5% (Table 1, Figure 6).

In general, IgA is the second common antibody in serum after IgG and is known to be present in saliva and serosity, in addition to blood. The fact that anti-pADPR IgA antibody in serum could be measured suggested a possibility that this measurement system can also be applied to saliva. Moreover, when anti-pADPR IgA and/or anti-H1 IgA is measured, the resulting value can be used as an index to detennine whether a test patient suffers from AD or SDAT.

### INDUSTRIAL APPLICABILITY

The method of the present invention uses the production of anti-poly ADP-ribose antibody and/or anti-histone H1 antibody as an index, and allows simple detection of these antibodies by an ELISA system in which poly ADP-ribose and/or histone H1 is solid-phased. For this reason, the present invention facilitates the detection and diagnosis of nervous system diseases.

Further, the present invention enables the detection and diagnosis of nervous system diseases as distinguished from SLE, which is an autoimmune disease, by calculating the IgG subclass G1/G2 ratio or by detecting anti-histone H1 antibody either alone or in combination with anti-poly ADP-ribose antibody.

Furthermore, the present invention enables the detection and diagnosis of nervous system diseases by using anti-pADPR IgA and/or anti-H1 IgA.

All references cited herein are hereby incorporated by reference in their entirety.

## Claims

1. A detection method for a nervous system disease, which comprises reacting poly ADP-ribose and/or histone H1 with a biological sample to detect an antibody against poly ADP-ribose and/or an antibody against histone H1.

2. The method according to claim 1, wherein the biological sample is a body fluid.

3. The method according to claim 1, wherein the biological sample is at least one selected from the group consisting of blood, saliva, serosity and lymph.

4. The method according to claim 1, wherein the biological sample is serum.

5. The method according to claim 1, wherein the nervous system disease is Alzheimer's disease or senile dementia of the Alzheimer's type.

6. The method according to claim 1, wherein the antibody detection is accomplished by using the ratio between IgG1 and IgG2 as an index.

7. The method according to claim 1, wherein the antibody detection is accomplished by using the value of IgG or IgA as an index.

8. A diagnostic method for a nervous system disease, which comprises reacting poly ADP-ribose and/or histone H1 with a biological sample to detect an antibody against poly ADP-ribose and/or an antibody against histone H1.

9. The method according to claim 8, wherein the biological sample is a body fluid.

10. The method according to claim 8, wherein the biological sample is at least one selected from the group consisting of blood, saliva, serosity and lymph.

11. The method according to claim 8, wherein the biological sample is serum.

12. The method according to claim 8, wherein the nervous system disease is Alzheimer's disease or senile dementia of the Alzheimer's type.

13. The method according to claim 8, wherein the antibody detection is accomplished by using the ratio between IgG1 and IgG2 as an index.

14. The method according to claim 8, wherein the antibody detection is accomplished by using the value of IgG or IgA as an index.

15. A diagnostic or detection kit for a nervous system disease, which comprises poly ADP-ribose and/or histone H1.

16. The kit according to claim 15, wherein the nervous system disease is Alzheimer's disease or senile dementia of the Alzheimer's type.

17. A diagnostic or detection plate for a nervous system disease, which comprises poly ADP-ribose and/or histone H1 immobilized on a solid phase.

18. The plate according to claim 17, wherein the nervous system disease is Alzheimer's disease or senile dementia of the Alzheimer's type.

19. A method for solid-phasing histone H1 onto a solid phase, which comprises diluting histone H1 with a solution of 0.25 M NaCl and 25 mM Tris buffer (pH 7.4) and then immobilizing the same on the solid phase.
